(19) Europäisches Patentamt
European Patent Office
Office européen des brevets

(11) Veröffentlichungsnummer: **0 270 970 B1**

(12) # EUROPÄISCHE PATENTSCHRIFT

(45) Veröffentlichungstag der Patentschrift: **18.03.92**

(51) Int. Cl.⁵: **C07C 201/12**, C07C 205/11, C07C 201/16

(21) Anmeldenummer: **87117668.1**

(22) Anmeldetag: **30.11.87**

Die Akte enthält technische Angaben, die nach dem Eingang der Anmeldung eingereicht wurden und die nicht in dieser Patentschrift enthalten sind.

(54) **Verfahren zur Herstellung von 3,5-Dichlor-2,4-difluornitrobenzol.**

(30) Priorität: **11.12.86 DE 3642333**

(43) Veröffentlichungstag der Anmeldung:
**15.06.88 Patentblatt 88/24**

(45) Bekanntmachung des Hinweises auf die Patenterteilung:
**18.03.92 Patentblatt 92/12**

(84) Benannte Vertragsstaaten:
**CH DE FR GB IT LI**

(56) Entgegenhaltungen:
**EP-A- 0 052 833**
**US-A- 3 294 629**

**CHEMICAL ABSTRACTS, Band 102, Nr. 11, 18. März 1985, S. 546, Zusammenfassung Nr. 95355f, Columbus, Ohio, US; T. TANABA: "Preparation of tetrachloronitrobenzonitriles and related compounds"**

**RESEARCH DISCLOSURE, Juli 1985, Nr. 255, Emsworth, Hampshire, GB**

(73) Patentinhaber: **BAYER AG**
**W-5090 Leverkusen 1 Bayerwerk(DE)**

(72) Erfinder: **Blank, Heinz Ulrich, Dr.**
**Am Geusfelde 35**
**W-5068 Odenthal(DE)**
Erfinder: **Ritzer, Edwin, Dr.**
**Burbachstrasse 31**
**W-5093 Burscheid(DE)**

EP 0 270 970 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die vorliegende Erfindung betrifft ein Verfahren zur Herstellung von 3,5-Dichlor-2,4-difluor-nitrobenzol durch Umsetzung von 2,3,4,5-Tetrachlor-nitorbenzol mit Fluoriden der Alkalimetalle in Dimethylsulfoxid (DMSO) oder einem DMSO-haltigen Lösungsmittelgemisch.

Der Austausch von aktivierten Chlor-substituenten in aromatischen Verbindungen mit Hilfe von Kaliumfluorid in aprotischen Lösungsmitteln ist grundsätzlich bekannt. Als aprotisches Lösungsmittel ist Dimethylsulfoxid (DMSO) sehr früh in Betracht gezogen worden. Jedoch wird immer von Zersetzungsreaktionen des DMSO berichtet, die zu übelriechenden, Thiomethyl-haltigen Nebenprodukten führen. (J. Am. Chem. Soc. 78, (1956), 6034-6037). Zur Verringerung dieser Geruchsbelästigung wird eine Behandlung der Reaktionsprodukte nach der Cl-F-Austauschreaktion mit Permanganat empfohlen. In Chem. and Ind. 1962, 1328-1329 wird daher der Ersatz von DMSO durch Dimethylsulfon ($DMSO_2$) vorgeschlagen.

Es hat nicht an Versuchen gefehlt, die relativ hohen Temperaturen für den Cl-F-Austausch, die zu Zersetzungen führen, zur erniedrigen. So wird in DE-OS 2 938 939 vorgeschlagen, Chlornitrobenzol mit KF in Gegenwart eines Phasentransfer-Katalysators bei Temperaturen von nicht höher als 200° C umzusetzen.

Des weiteren ist versucht worden, den negativen Einfluß von absorptiv am KF gebundenen Wasser auf die Reaktionszeit und die Reaktionstemperatur auszuschließen, indem man sprühgetrocknetes KF einsetzte (Chemistry Letters 1981, 761-764).

Bei höher mit Chlor substituierten Nitrobenzolen treten zu den genannten Schwierigkeiten auf Grund der Zersetzlichkeit des DMSO bei den erforderlichen Temperaturen noch die Schwierigkeiten wegen der Vielzahl von Substituenten, die unkontrollierte Nebenreaktionen befürchten lassen.

So wird in US-3 294 629 3,5-Dichlor-2,4-difluor-nitrobenzol aus 2,3,4,5-Tetrachlor-nitrobenzol und KF in DMSO in einer siebenstündigen Reaktion bei 110° C in nur 34 %iger Ausbeute erhalten. Auch in der Veröffentlichung in Gunma Journal of Liberal Arts and Sciences (Universität Gunma, Japan)18 (1984), 55-66 wird der Versuch unternommen, das aprotische Lösungsmittel Dimethylformamid (DMF) durch DMSO zu ersetzen, weil DMSO eine noch stärkere Polarität und einen noch höheren Siedepunkt als DMF hat. Die Umsetzung von 2,3,4,5-Tetrachlor-nitrobenzol in DMSO wurde mit KF durchgeführt, das durch Feinpulverisieren und wiederholtes Rösten entwässert worden war. Die Ausbeute in einer 4 stündigen Reaktion unter Rückfluß (ca. 189° C) betrug nur 14 %, während sie in einer 7 stündigen Reaktion unter Rückfluß in DMF (ca. 153° C) immerhin 49 % betrug; die allgemeine Beschreibung dieser Veröffentlichung nennt sogar 59 % Ausbeute in DMF. Das zugehörige Abstract in C.A. 102 (1985), 95 355 f ist insoweit falsch, als es diese Ausbeute von 59 % der Verwendung von DMSO (statt DMF) zuordnet.

Auch in EP 52 833 wird der Cl-F-Austausch an 2,3,4,5-Tetrachlor-nitrobenzol mit KF in DMF durchgeführt, wobei auf äußersten Wasserausschluß geachtet wird: KF wird durch mehrstündiges Glühen bei 600° C getrocknet, und DMF wird mit $P_2O_5$ und anschließend mit Hilfe eines Molekularsiebs getrocknet. Hierbei erhält man in einer 15 stündigen Reaktion bei 140° C eine Ausbeute von 62 % der theoretischen Ausbeute. Im Research Disclosure RD 25 517 (Juli 1985) wird für die gleiche Reaktion Dimethylsulfon als Lösungsmittel eingesetzt und in einer 8 stündigen Reaktion bei 135° C eine Ausbeute von 84 % erhalten.

Ein geringfügig anderes Verfahren zur Herstellung von 3,5-Dichlor-2,4-difluor-nitrobenzol wird in DE-OS 3 435 889 beschrieben. Hierbei werden in 2,3,4-Trichlor-nitrobenzol das 2- und das 4-Chloratom durch Fluor ersetzt, und das Reaktionsprodukt wird anschließend kernchloriert. Als Lösungsmittel für den Cl-F-Austausch wird DMF oder Tetramethylensulfon (Sulfolan) verwendet, wobei darauf hingewiesen wird, daß DMSO nach Literaturangaben eine Ausbeute von nur 23 % der theoretischen Ausbeute ermöglicht.

Die bislang veröffentlichten Angaben lassen DMSO bei diesen Reaktionen als ein nicht geeignetes Lösungsmittel erscheinen, da die Ausbeuten äußerst unbefriedigend sind und mit dem Auftreten unerwünschter Nebenreaktionen gerechnet werden muß. Dies ist umso bedauerlicher, als DMSO eine Reihe vorteilhafter Eigenschaften besitzt, deren Nutzung wünschenswert ist:

a) DMSO besitzt nicht die Toxizität von anderen geeigneten Lösungsmitteln, beispielsweise die von DMF. Während beispielsweise DMSO in der Verabreichung von Arzneimittel benutzt wird, ist DMF arbeitshygienisch bedenklich, so daß der 1958 festgelegte MAK-Wert mit 20 ppm recht niedrig angesetzt ist.

b) DMSO kann bei der wäßrigen Aufarbeitung von Reaktionsgemischen mit geradkettigen, verzweigten oder cyclischen Aliphaten extrahiert und so gut in einer wasserfreien Form wiedergewonnen werden, ohne daß eine kosten- und zeitaufwendige Destillation von Wasser/DMSO-Gemischen mit anschließender Absolutierung des DMSO notwendig ist. Prinzipiell ist jedoch auch eine Trocknung von DMSO durch Destillation in Betracht zu ziehen.

c) Eine weitere positive Eigenschaft des DMSO hat sich erst bei dar Entwicklung des erfindungsgemäßen Verfahrens herausgestellt: die bislang für nötig gehaltenen und weiter oben erwähnten sehr

drastischen Trocknungsmethoden der Einsatzstoffe, insbesondere des Alkalifluorids, sind in DMSO überflüssig, so daß eine normale Handelsqualität, beispielsweise von KF, ausreicht.

Es wurde nun überraschend gefunden, daß entgegen den Angaben der Literatur DMSO bei der Herstellung von 3,5-Dichlor-2,4-difluor-nitrobenzol erfolgreich eingesetzt werden kann, wenn unter den Bedingungen des erfindungsgemäßen Verfahrens gearbeitet wird.

Die Erfindung betrifft demnach ein Verfahren zur Herstellung von 3,5-Dichlor-2,4-difluor-nitrobenzol durch Umsetzung von 2,3,4,5-Tetrachlor-nitrobenzol mit Fluoriden der Alkalimetalle bei erhöhter Temperatur in einem polaren, aprotischen Lösungsmittel, das dadurch gekennzeichnet ist, daß man als Lösungsmittel Dimethylsulfoxid (DMSO) oder ein DMSO-haltiges Gemisch, weiches mindestens 50 Gew.-% DMSO enthält, einsetzt und bei einer Temperatur von 60-160 °C und einer Reaktionszeit von 20-0,2 Stunden arbeitet, wobei Temperatur und Zeit durch die Beziehung T(°C) = A-33 log t(h) mit Werten für A von 100-135 verknüpft sind.

Das im erfindungsgemäßen Verfahren einzusetzende 2,3,4,5-Tetrachlor-nitrobenzol ist bekannt und kann durch Nitrierung von Tetrachlorbenzol hergestellt werden.

Als Fluoride verwendet man vorzugsweise die Fluoride der schweren Alkalimetalle. Beispielsweise seien Kaliumfluorid, Rubidiumfluorid oder Cäsiumfluorid sowie Gemische hieraus genannt. In bevorzugter Weise wird Kaliumfluorid eingesetzt. Selbstverständlich kann dem Kaliumfluorid ein an sich bekannter Zusatz von Kaliumchlorid, beispielsweise 0,5-20 Gew.-%, bezogen auf die Menge des KF, beigegeben werden. In bevorzugter Weise wird ohne einen solchen Zusatz gearbeitet.

Im erfindungsgemäßen Verfahren können ferner ein oder mehrere Phasentransfer-Katalysatoren, beispielsweise in einer Menge von 0,3-30 Gew.-%, bezogen auf das umzusetzende 2,3,4,5-Tetrachlor-nitrobenzol, zugesetzt werden. Solche Phasentransfer-Katalysatoren sind beispielsweise Tetrabutylammoniumbromid, Trimethylphenylammoniumchlorid, Triethylbenzylammoniumchlorid, Trimethylbenzylammoniumchlorid, Hexadecyltributylphosphoniumbromid, Kronenether (18-Krone-6).

Das Lösungsmittel DMSO kann als solches oder als Gemisch mit einem anderen inerten Lösungsmittel eingesetzt werden. Das andere inerte Lösungsmittel kann in einer Menge von bis zu 50 Gew.-%, bezogen auf das Gesamtgewicht von DMSO und diesem anderen Lösungsmittel, eingesetzt werden. Geeignete Lösungsmittel dieser Art sind Benzol, Toluol, Chlorbenzol, Dichlorbenzol, Trichlorbenzol oder Tetrachlorbenzol.

In bevorzugter Weise wird DMSO allein eingesetzt.

Für den Fall, daß der Siedepunkt dieses anderen Lösungsmittels bei Normaldruck niedriger als die angestrebte Reaktionstemperatur liegt, kann auch bei erhöhtem Druck, beispielsweise bei 1,5-10 bar, gearbeitet werden. Durch das Arbeiten bei vermindertem Druck kann man die Temperatur sehr gut durch das Einstellen der entsprechenden Siedegleichgewichte regulieren ("Siedekühlung").

Das erfindungsgemäße Verfahren wird bei einer Temperatur von 60-160 °C, bevorzugt 80-140 °C, besonders bevorzugt 95-125 °C durchgeführt. Die Reaktionszeit beträgt 20-0,2 Stunden, und die Temperatur und die Zeit sind durch die Beziehung T(°C) = A-33 log t(h) verknüpft, worin A Werte von 100-135, bevorzugt 115-130 annimmt.

Das Verhältnis von 2,3,4,5-Tetrachlor-nitrobenzol zu Kaliumfluorid zu DMSO kann im erfindungsgemäßen Verfahren von 1:2,2:2,0 bis 1:5:24, bevorzugt von 1:2,3:2,4 bis 1:4,0:10, besonders bevorzugt von 1:2,4:2,8 bis 1:3,2:4,6 betragen. Dies ist ein gegenüber den bekannten Verfahren viel günstigerer Wert und ist in Richtung auf höhere Feststoffgehalte in der Suspension in DMSO nur durch die abnehmende Rührbarkeit der Suspension begrenzt. Hierbei wird im erfindungsgemäßen Verfahren eine gegenüber den bekannten Verfahren erheblich gesteigerte Raum-Zeit-Ausbeute erreicht; die hohen Stoffkonzentrationen ermöglichen dazu noch beträchtliche Energieeinsparungen.

Das DMSO kann erfindungsgemäß ohne besondere Vorbehandlung eingesetzt werden, da der Feuchtigkeitsgehalt im allgemeinen so gering ist, daß es zu keinem Zusammenklumpen des Alkalifluorids kommt. Sollte nur ein wasserhaltiges DMSO zur Verfügung stehen, kann es durch übliche Trocknung, beispielsweise über Phosphorpentoxid oder anderen bekannten Trockenmitteln, getrocknet werden. Eine weitere Möglichkeit besteht darin, etwa vorhandenes Wasser nach Zusatz von Toluol oder einem anderen Azeotropbildner azeotrop abzudestillieren oder aber die Trocknung von DMSO nur durch Destillation durchzuführen. Es hat sich gezeigt, daß der erfindungsgemäße Einsatz von DMSO mit seinen beschriebenen günstigen Eigenschaften für die Durchführung der C1-F-Austauschreaktion von größerer Bedeutung ist als der Feinheitsgrad der eingesetzten Alkalifluoride.

Das Alkalifluorid kann in verschiedenen Trocknungsgraden eingesetzt werden. So ist es möglich, das Alkalifluorid durch Sprühtrocknung vorzubehandeln. Weiterhin ist es möglich, durch längeres sehr scharfes Trocknen, beispielsweise einige Stunden bei bis zu 600 °C, vorzubehandeln. Eine noch weitere Möglichkeit besteht darin, das Alkalifluorid im DMSO vorzulegen und nach Zusatz von beispielsweise Toluol oder einem

anderen Azeotropbildner das im System (im Alkalifluorid oder im DMSO) vorhandene Wasser azeotrop abzudestillieren. Es hat sich jedoch gezeigt, daß im erfindungsgemäßen Verfahren das Erfordernis des Feuchtigkeitsausschlusses bei weitem nicht so extrem eingehalten werden muß wie bei den Verfahren des Standes der Technik. So ist es ausreichend, das Alkalifluorid im Trockenschrank bei 200 °C/200 Torr zu trocken. Das erfindungsgemäße Verfahren ist sogar bei Verwendung von handelsüblichen trocken Alkalifluoriden durchführbar.

Im allgemeinen werden demnach das DMSO oder das DMSO-haltige Lösungsmittelgemisch und das Alkalifluorid vorgelegt und, falls dies erforderlich ist, einer Trocknung durch Azeotropdestillation unterworfen. Hierbei wird vielfach bereits die angestrebte Reacktionstemperatur erreicht. Das 2,3,4,5-Tetrachlor-nitrobenzol wird dann zur Suspension des Alkalifluorids in DMSO oder dem DMSO-haltigen Gemisch gegeben; diese Zugabe kann nach Erreichen der Reaktionstemperatur oder bereits vor oder während der Aufheizphase erfolgen. Die Reaktionszeit beginnt mit dem Erreichen der Reaktionstemperatur, mit der sie im oben angegebenen Zusammenhang steht. Bei größern Ansätzen im technischen Umfang kann es wünschenswert sein, den Ausgangsstoff in Portionen zuzugeben; in jedem Fall benötigt die Zugabe einer größeren Menge des Ausgangsstoffes eine angemessene, dem Fachmann aus der chemischen Verfahrenstecknik bekannte Zeit. In einem solchen Falle wird der Begin der Reaktionszeit auf das Ende der Zugabe gesetzt.

Nach Beendigung der Cl-F-Austauschreaktion kann das entstandene Reaktionsgemisch in verschiedener Weise aufgearbeitet werden. So werden im allgemeinen nach Abkühlen des Reaktionsgemisches zunächst die anorganischen Salze (Alkalimetallfluoride/-chloride) abgetrennt, z.B. abfiltriert oder abzentrifugiert. Die weitere Aufarbeitung kann dann durch Destillation, Extraktion oder durch andere physikalische Trennverfahren wie Säulenchromatographie durchgeführt werden. Beispielsweise kann die Lösung des 3,5-Dichlor-2,4-difluor-nitrobenzol in DMSO mit Wasser versetzt werden, wobei sich das 3,5-Dichlor2,4-difluornitrobenzol abscheidet, abgetrennt werden kann und einer gegebenenfalls weiteren Reinigung zugeführt werden kann.

In einer besonderen Ausgestaltung des erfindungsgemäßen Verfahrens wird die DMSO-Lösung diskontinuierlich oder kontinuierlich, in bevorzugter Weise kontinuierlich, mit einem oder mehreren, vorzugsweise gesättigten, offenkettigen oder cyclischen aliphatischen Kohlenwasserstoffen extrahiert. Hierbei tritt das Reaktionsprodukt in die Phase des aliphatischen Kohlenwasserstoffes über und kann daraus durch Abdampfen des aliphatischen Kohlenwasserstoffes gewonnen werden und gegebenenfalls einer weiteren Feinreinigung zugeführt werden. Das durch Extraktion vom Reaktionsprodukt befreit DMSO kann als solches ohne weitere Aufarbeitung dem nächsten Reaktionsansatz im erfindungsgemäßen Verfahren zugeführt werden. Geeignete Extraktionsapparate für diese spezielle Verfahrensvariante sind dem Fachmann bekannt. Als gesättigte, offenkettige oder cyclische Aliphaten seien solche genannt, deren Siedepunkt mindestens 30 °C beträgt, wie Pentan, Hexan, Octan, Decan, Dodecan, Hexadodecan, Cyclohexan, Methylcyclopentan, Methylcyclohexan, Isooktan, sowie die aliphatischen Destillationsschnitte Petrolether mit Siedebereichen 30-50 °C, ca. 40 °C, 40-60 °C, 60-70 °C, 40-80 °C, Leichtbenzin (60-95 °C), Ligroin (80-110 °C), Lötbenzin (60-140 °C), Waschbenzin (100-140 °C) u.ä..

Beim Verwässern der DMSO-Lösung und anschließender Extraktion dieser DMSO-Wasserlösungen mit anderen organischen Lösungsmitteln als den obengenannten, beispielsweise mit Methylenchlorid, Chloroform oder Toluol kommt es zu langsamer Ausbildung der Phasengrenze, die außerdem visuell nur schlecht erfaßbar sein kann. Solche Probleme bei dieser Art von Extraktion der Umsetzungsprodukte von 2,3,4,5-Tetrachlor-nitrobenzol mit KF in DMSO können nur umgangen werden, wenn man auf das Verwässern der DMSO-Phase und Extraktion mit diesen anderen Lösungsmitteln verzichtet.

Das erfindungsgemäß herstellbare 3,5-Dichlor-2,4-difluor nitrobenzol ist ein wichtiges Zwischenprodukt zur Herstellung insektizider Wirkstoffe (US 3 294 629; EP 52 833).

Für die Weiterverarbeitung zu solchen Wirkstoffen wird das 3,5-Dichlor-2,4-difluor-nitrobenzol vielfach vorher in das zugehörige substituierte Anilin durch Hydrierung umgewandelt. So kann neben anderen dem Fachmann bekannten Hydrierverfahren die aus der beschriebenen Extraktion gewonnene Lösung der Nitroverbindung in einem der genannten aliphatischen Kohlenwasserstoffe mit dem Hydrierkatalysator (beispielsweise Platinmetalle auf Kohle, Raney-Nickel oder ähnlichen) versetzt werden und durch Zugabe von Wasserstoff hydriert werden. Nach beendeter Wasserstoffaufnahme trennt man den Katalysator ab und arbeitet in üblicher Weise auf oder fällt das 3,5-Dichlor-2,4-difluor-anilin als Salz, z.B. mit HCl-Gas in Form des Hydrochlorids aus und trennt es in dieser Form vom Lösungsmittel ab. Selbstverständlich ist nach vorrangegangener Gewinnung des 3,5-Dichlor-2,4-difluor-nitrobenzols auch eine Hydrierung in anderen Lösungsmitteln, beispielsweise einem anderen aliphatischen Kohlenwasserstoff, in Aromaten, Alkoholen usw., möglich. Ebenso ist grundsätzliche eine Reduktion der Nitrogruppe mit Eisen/Säure zur Anilingruppe möglich.

Außer dem angestrebten Cl-F-Austausch findet als Nebenreaktion vielfach ein $NO_2$-F-Austausch statt,

bei dem 2,3,4,5-Tetrachlor-fluorbenzol entsteht (vgl. Formelschema zu den Beispielen 3-14:I→III). Nach der oben genannten Hydrierung der Nitroverbindung zum zugehörigen Anilin und Ausfällung mit HCl als Hydrochlorid bleibt das 2,3,4,5-Tetrachlor-fluorbenzol in Lösung und kann aus dieser Lösung in bekannter Weise rein dargestellt und einer eigenen Verwendung zugeführt werden. Selbstverständlich kann das 2,3,4,5-Tetrachlor-fluorbenzol auch durch Säulenchromatographie oder durch die üblichen physikalischen Trennverfahren, z.B. durch Destillation von der Nitroverbindung oder dem zugehörigen Amin in reiner Form abgetrennt werden.

Eine Abtrennung dieser Nebenprodukte erfolgt gegebenenfalls zweckmäßigerweise erst nach Umsetzung der Aniline zu einem Folgeprodukt.

Beispiel 1

130 g 2,3,4,5-Tetrachlor-nitrobenzol (0,5 mol) wurden mit 75,5 g KF (1,3 mol) in 130 g DMSO suspensiert und 4 Stunden auf 110 °C erwärmt. Nach dem Abkühlen auf Raumtemperatur wurde das KF/KCl-Feststoffgemisch über eine Nutsche abfiltriert und zweimal mit je 30 ml DMSO gewaschen. Die erhaltene DMSO-Lösung wurde kontinuierlich mit kaltem Hexan in einem 300 ml-Rotations-Perforator nach Ludwig zur Flüssig-Flüssig-Extraktion mit spezifisch leichteren Lösungsmitteln (DE-AS 2 221 554, Firma Normag) extrahiert. Der Perforator wurde hierbei von außen mit Wasser gekühlt, um eine Erwärmung des Extraktionsgutes durch das zurückfließende n-Hexan und dadurch einen Übergang größerer Mengen DMSO in die Hexanphase zu vermeiden. Aus der Hexanphase wurde 3,5-Dichlor-2,4difluor-nitrobenzol in 78 %iger Ausbeute, bezogen auf die theoretische Ausbeute, gewonnen. Die Hexanphase enthielt weiterhin nach gaschromatographischer Bestimmung 10%, bezogen auf die theoretische Menge, 2,3,4,5-Tetrachlor-fluorbenzol.

Die Trennung von 3,5-Dichlor-2,4-difluor-nitrobenzol und 2,3,4,5-Tetrachlor-fluorbenzol kann über eine kurze Chromatographie-Säule, gefüllt mit für Säulenchromatographie handelsüblichem Kieselgel, erfolgen.

Nach dem Aufgeben des Rohprodukts, gelöst in aliphatischen Kohlenwasserstoffen (z.B. Hexen), eluiert man dan 2,3,4,5-Tetrachlorfluorbenzol vollständig, indem man ebenfalls aliphatische Kohlenwasserstoffe als Laufmittel wählt; anschließend wechselt man zu polareren Laufmitteln (z.B. $CH_2Cl_2$, $CHCH_3$ oder anderen) und erhält so das 3,5-Dichlor-2,4-difluor-nitrobenzol.

Durch Entfernen des Lösungsmittels erhält man die jeweiligen reinen Stoffe und kann sie auf übliche Weise (Kristallisation, Destillation) noch einer zusätzlichen Reinigungsstufe unterwerfen.

Beispiel 2

Es wurde wie in Beispiel 1 gearbeitet, jedoch wurde die Reaktionstemperatur auf 100 °C festgesetzt. Man erhielt 84 %, bezogen auf die theoretische Ausbeute, an 3,5-Dichlor-2,4-difluor-nitrobenzol. Die Hexanphase enthielt laut gaschromatographischer Bestimmung 9 %, bezogen auf die theoretische Menge, an 2,3,4,5-Tetrachlor-fluorbenzol.

Besipiele 3-14

1 mol 2,3,4,5-Tetrachlor-nitrobenzol wurde mit dem in der Tabelle angegebenen Molmengen KF und 260 g DMSO bei den in der Tabelle angegebenen Temperaturen und Zeiten gerührt. Nach Beendigung der Reaktion trennte man die anorganische Salze (KF und KCl) ab, wuscht mit DMSO nach und untersuchte das Filtrat gaschromatographisch.

Für den Fall der $H_2O$-Entfernung durch azeotrope Trocknung mit Toluol wurden KF, gegebenenfalls der Phasentransferkatalysator und das DMSO mit Toluol versetzt, 4 Stunden am Wasserabscheider erhitzt und anschließend durch Destillation weitgehend vom Toluol befreit (das Toluol kann jedoch nach der Entwässerung auch als Lösungsmittelanteil im Ansatz verbleiben). Anschließend wurde die Reaktionstemperatur eingestellt und der Ausgangsstoff zugegeben.

In anderen Fallen wurde KF über Nacht bei 250 °C/200 mbar entwässert.

In noch anderen Fällen wurde handelsübliches trockenes oder sprühgetrocknetes KF eingesetzt.

Die hierbei gefundene Werte für Umsatz, Ausbeute und Vorhandensein verschiedener Verunreinigungen sind in der folgenden Tabelle angegeben. Die mit römischen Ziffern bezeichneten Produkte in der Tabelle können formelmäßig wie folgt dargestellt werden:

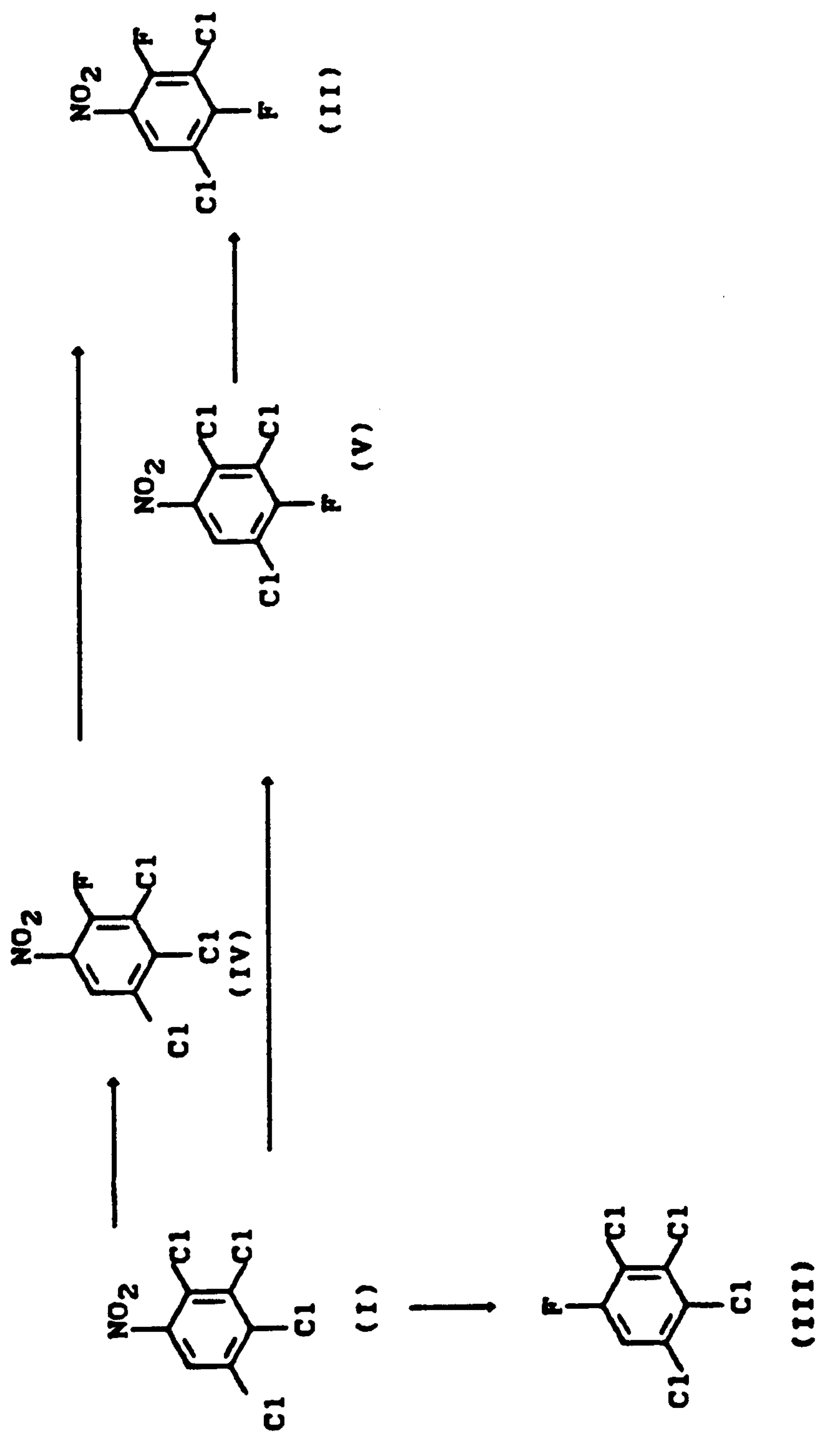

(II) (V) (IV) (I) (III)

**Tabelle 1** (Beispiele 3-14): Umsetzung von I mit KF in DMSO zu II

| Nr. | KF (Mol) | T (°C) | t (h) | Phasentransfer-Katalysator | Umsatz/Ausbeute (%) | Verhältnis II:III | IV+V (%) | Bemerkungen |
|---|---|---|---|---|---|---|---|---|
| 3 | 3* | 100 | 4 | - | 99/78 | 1:0,10 | - | - |
| 4 | 3** | 100 | 4 | $N(Butyl)_4Br$ | 100/85 | 1:0,12 | - | - |
| 5 | 3** | 100 | 4 | $N(Butyl)_4Br$ | 98/77 | 1:0,12 | - | DMSO: Toluol= 3:1 |
| 6 | 3** | 100 | 4 | $N(Ethyl)_3(phenyl)Cl$ | 99/75 | 1:0,11 | - | - |
| 7 | 3** | 100 | 4 | $P(Butyl)_3(C_{16}H_{33})Br$ | 99/88 | 1:0,11 | - | - |
| 8 | 3*** | 80 | 16 | - | 99/80 | 1:0,08 | - | - |
| 9 | 3*** | 100 | 4 | - | 99/84 | 1:0,10 | - | - |
| 10 | 2,2*** | 100 | 4 | - | 98/80 | 1:0,12 | 5 | - |
| 11 | 3*** | 120 | 1 | - | 100/82 | 1:0,14 | - | - |
| 12 | 3*** | 120 | 2 | - | 100/80 | 1:0,16 | - | - |
| 13 | 3*** | 100 | 4 | - | 99/82 | 1:0,12 | - | KF mit 2% KCl |
| 14 | 3*** | 100 | 4 | - | 98/77 | 1:0,11 | - | KF mit 20% KCl |

* Handelsware, Entfernung von $H_2O$-Spuren durch Azeotropdestillation mit Toluol aus der Suspension in DMSO vor dem Zusatz von I

** sprühgetrocknet und Azeotropdestillation

*** sprühgetrocknet und Vakuumtrocknung (200°C/200 Torr)

Beispiele 15 - 27

In gleicher Weise wie bei den vorausgegangenen Beispielen wurde jeweils 0.1 Mol (26,1 g) I mit den in der Tabelle 2 angegebenen Mengen KF und Zusatzstoff in jeweils 150 ml DMSO umgesetzt. Tabelle 2

enthält weiter die Reaktionsbedingungen und die Ergebnisse.

Ausnahmen: Im Beispiel 21 wurden nur 100 ml DMSO eingesetzt. Im Beispiel 26 wurden 39,2 g (0,15 Mol) I umgesetzt.

Die Beispiele zeigen, daß ein erhöhter Trocknungsaufwand und die dafür erforderliche Behandlungszeit sowie der Zusatz von Phasentransferkatalysatoren entbehrlich sind.

**Tabelle 2** (Beispiele 15-27): Umsetzung von I mit KF in DMSO zu II

| Nr. | KF (g) | Zusatzstoff (g) | Molverhältnis KF:I:Zusatz | T (°C) | t (h) | Umsatz (%) | Ausbeute (%) II und IV + V |
|---|---|---|---|---|---|---|---|
| 15 | 18* | - | 3,1:1:0 | 100 | 4 | >99 | 84/- |
| 16 | 18* | 5,1 $P(C_4H_9)_3(C_{16}H_{33})Br$ | 3,1:1:0,1 | 100 | 4 | >99 | 84/- |
| 17 | 18* | 3,1 $N(C_4H_9)_4Br$ | 3,1:1:0,1 | 100 | 4 | >99 | 84/- |
| 18 | 18* | 2,1 $N(C_2H_5)_3(C_6H_5)Cl$ | 3,1:1:0,1 | 100 | 4 | 99 | 84/- |
| 19 | 18** | 3,2 $N(C_4H_9)_4Br$ | 3,1:1:0,1 | 100 | 4 | 97 | 76/9 |
| 20 | 18*** | - | 3,1:1:0 | 120 | 1 | >99 | 82/- |
| 21 | 12,8*** | - | 2,2:1:0 | 100 | 4 | >99 | 79/6 |
| 22 | 12,8*** | - | 2,2:1:0 | 100 | 6 | >99 | 79/6 |
| 23 | 18*** | 5 KCl | 3,1:1:0,7 | 100 | 4 | 96 | 76/8 |
| 24 | 18**** | 0,5 KCl | 3,1:1:0,07 | 100 | 4 | >99 | 83/- |
| 25 | 27**** | - | 3,1:1:0 | 100 | 4 | >99 | 78/6 |
| 26 | 18**** | - | 3,1:1:0 | 80 | 16 | 98 | 80/5 |
| 27 | 18**** | - | 3,1:1:0 | 100 | 4 | 98 | 81/4 |

* Azeotrop-Entwässerung mit 100 ml Toluol und anschließendes Abdestillieren des Toluols

** wie bei *, jedoch verblieben 50 ml Toluol im Reaktionsansatz

*** handelsübliches trockenes KF

**** KF bei 250°C/200 mbar über Nacht getrocknet

Beispiel 28

Die Umsetzung des 2,3,4,5-Tetrachlor-nitrobenzols mit KF in DMSO wurde, wie in Beispiel 2 beschrieben, durchgeführt. Nach dem Abtrennen und Waschen der Salze wurden jedoch 500 ml Wasser zugesetzt und das Produkt fünfmal mit je 300 ml Methylenchlorid bzw. Chloroform aus der DMSO/$H_2O$-Phase extrahiert. Die vereinigten Methylenchlorid- bzw. Chloroformphasen wurden nun 5 mal mit 300 ml Wasser gewaschen und nach dem Trocknen über $MgSO_4$ bis zur völligen Entfernung von $CH_2Cl_2$ bzw. $CHCl_3$ eingeengt. Die Ausbeute beträgt 68 % an 3,5-Dichlor-2,4-difluor-nitrobenzol, bezogen auf die theoretische Menge.

## Patentansprüche

1. Verfahren zur Herstellung von 3,5-Dichlor-2,4-difluor-nitrobenzol durch Umsetzung von 2,3,4,5-Tetrachlor-nitrobenzol mit Fluoriden der Alkalimetalle bei erhöhter Temperatur in einem polaren, aprotischen Lösungsmittel, dadurch gekennzeichnet, daß man als Lösungsmittel Dimethylsulfoxid (DMSO) oder ein DMSO-haltiges Gemisch, welches mindestens 50 Gew.-% DMSO enthält, einsetzt und bei einer Temperatur von 60-160 °C und einer Reaktionszeit von 20-0,2 Stunden arbeitet, wobei Temperatur und Zeit durch die Beziehung T(°C) = A-33 log t(h) mit Werten für A von 100-135 verknüpft sind.

2. Verfahren nach Anspruch 1, dadurch gekennzeichnet, daß als Lösungsmittel DMSO genommen wird.

3. Verfahren nach Ansprüchen 1 und 2, dadurch gekennzeichnet, daß als Alkalifluorid Kaliumfluorid eingesetzt wird.

4. Verfahren nach Ansprüchen 1 bis 3, dadurch gekennzeichnet, daß das Kaliumfluorid ohne besondere Vorbehandlung eingesetzt wird.

5. Verfahren nach Ansprüchen 1 bis 4, dadurch gekennzeichnet, daß bei einer Temperatur von 80-140 °C gearbeitet wird.

6. Verfahren nach Ansprüchen 1 bis 5, dadurch gekennzeichnet, daß bei einer Temperatur von 95-125 °C gearbeitet wird.

7. Verfahren nach Ansprüchen 1 bis 6, dadurch gekennzeichnet, daß für A Werte von 115-130 eingesetzt werden.

8. Verfahren nach Ansprüchen 1 bis 7, dadurch gekennzeichnet, daß zur Aufarbeitung das Reaktionsprodukt mit einem oder mehreren vorzugsweise gesättigten, offenkettigen oder cyclischen aliphatischen Kohlenwasserstoffen mit einem Siedepunkt von mindestens 30 °C aus dem DMSO oder dem DMSO-haltigen Lösungsmittelgemisch mit einem Gehalt von mindestens 50 Gew.-% an DMSO durch Extraktion abgetrennt wird und durch physikalische Trennmethoden aus dem Extraktionsmittel gewonnen wird.

## Claims

1. Process for the preparation of 3,5-dichloro-2,4-difluoro-nitrobenzene by reacting 2,3,4,5-tetrachloronitrobenzene with fluorides of the alkali metals at elevated temperature in a polar, aprotic solvent, characterized in that the solvent employed is dimethyl sulphoxide (DMSO) or a DMSO-containing mixture which contains at least 50% by weight of DMSO, and the process is carried out at a temperature of 60-160 °C and a reaction time of 20-0.2 hours, the temperature and time being linked by the relationship T(°C) = A-33 log t(h), with values for A of 100-135.

2. Process according to Claim 1, characterized in that the solvent used is DMSO.

3. Process according to Claims 1 and 2, characterized in that the alkali metal fluoride employed is potassium fluoride.

4. Process according to Claims 1 to 3, characterized in that the potassium fluoride is employed without particular pretreatment.

5. Process according to Claims 1 to 4, characterized in that it is carried out at a temperature of 80-140 ° C.

6. Process according to Claims 1 to 5, characterized in that it is carried out at a temperature of 95-125 ° C.

7. Process according to Claims 1 to 6, characterized in that values of 115-130 are employed for A.

8. Process according to Claims 1 to 7, characterized in that the reaction product is worked up by removal from the DMSO or DMSO-containing solvent mixture having a content of at least 50% by weight of DMSO by extraction with one or more, preferably saturated, open-chain or cyclic aliphatic hydrocarbons having a boiling point of at least 30 ° C, and is obtained from the extracting agent by physical separation methods.

**Revendications**

1. Procédé de préparation du 3,5-dichloro-2,4-difluoro-nitrobenzène par réaction du 2,3,4,5-tétrachloro-nitrobenzène avec des fluorures de métaux alcalins à chaud dans un solvant aprotonique polaire, caractérisé en ce que l'on utilise en tant que solvant le diméthylsulfoxyde (DMSO) ou un mélange contenant du DMSO en proportions d'au moins 50 % en poids, et on opère à une température de 60 à 160 ° C dans une durée de réaction de 20 à 0,2 h, la température et la durée devant satisfaire à la relation $T(°C) = A - 33 \log t(h)$ dans laquelle A a des valeurs de 100 à 135.

2. Procédé selon la revendication 1, caractérisé en ce que le solvant est le DMSO.

3. Procédé selon les revendications 1 et 2, caractérisé en ce que le fluorure alcalin est le fluorure de potassium.

4. Procédé selon les revendications 1 à 3, caractérisé en ce que l'on utilise le fluorure de potassium sans aucun traitement préalable particulier.

5. Procédé selon les revendications 1 à 4, caractérisé en ce que l'on opère à une température de 80 à 140 ° C.

6. Procédé selon les revendications 1 à 5, caractérisé en ce que l'on opère à une température de 95 à 125 ° C.

7. Procédé selon les revendications 1 à 6, caractérisé en ce que A a des valeurs de 115 à 130.

8. Procédé selon les revendications 1 à 7, caractérisé en ce que, pour l'isolement du produit de réaction, on le sépare du DMSO ou du mélange solvant contenant du DMSO à une teneur d'au moins 50 % en poids, par extraiction à l'aide d'un ou plusieurs hydrocarbures aliphatiques de préférence saturés, acycliques ou cycliques, ayant un point d'ébullition d'au moins 30 ° C, et on l'isole du liquide d'extraction par des techniques de séparation physiques.